# EUROPÄISCHE PATENTSCHRIFT

(11) **EP 0 701 804 B1**
(45) Veröffentlichungstag und Bekanntmachung des Hinweises auf die Patenterteilung: **07.04.1999**
(21) Anmeldenummer: 95113179.6
(22) Anmeldetag: 22.08.1995
(51) Int. Cl.: A61F 13/14, A61F 13/58

(54) **Nabelverband**
Umbilical dressing
Pansement ombilical

(30) Priorität: 15.09.1994 DE 9415018 U
(43) Veröffentlichungstag der Anmeldung: 20.03.1996
(73) Patentinhaber: Holthaus Medical GmbH & Co KG, 42899 Remscheid (DE)
(72) Erfinder: Holthaus, Andreas G., 42899 Remscheid (DE)
(74) Vertreter: Alber, Norbert, Dipl.-Ing.

(56) Entgegenhaltungen:
- EP-A- 0 420 780
- DE-B- 1 273 129
- FR-A- 1 155 814
- FR-A- 2 604 867

## Beschreibung

Die Erfindung betrifft einen Nabelverband, wie er insbesondere zur Versorgung eines noch nicht vollständig abgeheilten Nabels bei einem Neugeborenen verwendet wird.

Dabei werden entweder manuell auf den Nabel Kompressen aufgelegt, und dann mittels einer Fixierbinde, entweder bestehend aus einem endlich langen Streifen oder in Form eines endlosen Schlauches, befestigt.

Darüberhinaus ist es auch bereits bekannt, einen Nabelverband zu benutzen, bei dem die Kompresse bereits fest auf der Fixierbinde angeordnet ist, und auch die gleiche Breite wie die Fixierbinde besitzt, so daß diese auf zwei Seiten über die Kompresse hinausragt.

So zeigt etwa die deutsche Auslegeschrift 1 273 129 einen solchen Nabelverband, bei dem jedoch das eine Ende der Fixierbinde zweilagig und damit V-förmig ausgebildet ist, um zwischen diese beiden Lagen das gegenüberliegende Ende der Fixierbinde bei der Applikation einlegen zu können. Dies ist notwendig, um durch den für die Befestigung benutzten Klettverschluß nicht die Gefahr eines Kontaktes mit der Haut des Babys einzugehen, was aufgrund der harten Einzelteile des Klettverschlusses zu Verletzungen führen kann.

Dabei besteht das Problem darin, daß der Bauchumfang der Neugeborenen unterschiedlich groß ist, und darüberhinaus auch bei jedem einzelnen Baby in Abhängigkeit von der momentanen Nahrungsaufnahme stark schwankt. Dennoch soll der durch den Nabelverband aufgebrachte Druck auf den Nabel immer etwa gleich groß sein. Denn bei einem zu geringen Druck auf den Nabel kann dieser unerwünscht hervortreten und bei zu starkem Druck wird die Nahrungsaufnahme des Kindes behindert bzw. das Kind reagiert mit Erbrechen.

Davon ausgehend, soll das Risiko einer Infektion des noch nicht abgeheilten Nabels so gering wie möglich gehalten werden. Aus diesem Grund wird angestrebt, die verwendeten Materialien, die im sterilen Zustand angeliefert werden, so schnell wie möglich und unter so geringen Berührungen wie möglich auf dem Nabel aufbringen zu können.

Es ist daher die Aufgabe gemäß der Erfindung, einen Nabelverband zu schaffen, der einfach und schnell in der richtigen Art und Weise aufzubringen ist, dessen Kontaminationsrisiko möglichst gering ist und der einfach und kostengünstig in der Herstellung ist. Diese Aufgabe wird durch die kennzeichnenden Merkmale des Anspruches 1 gelöst. Vorteilhafte Ausführungsformen ergeben sich aus den Unteransprüchen.

Einerseits wird durch die spezifische Länge der Fixierbinde, die sich etwa einmal um den Bauchumfang des Babys erstreckt, ein Klemmeffekt, wie er beim mehrfachen Umwickeln aufgrund der immer stärker werdenden Reibung zwischen den einzelnen Lagen der applizierten Fixierbinde auftritt, vermieden. Andererseits ist die Fixierbinde so lang, daß der Bauchumfang immer mindestens einmal vollständig abgedeckt ist, so daß der Haftstreifen für die Applizierung immer nur auf der Fixierbinde angeordnet wird, und niemals, auch nur teilweise, auf der Haut des Babys. Dadurch ist es möglich, einen einfachen Klebestreifen mit normalem Klebstoff zu verwenden, da dieser weder mit der Haut des Babys in Berührung kommt, noch an einer Stelle der Fixierbinde appliziert wird, bei der eine Durchfeuchtung aufgrund des noch nicht abgeheilten Nabels zu befürchten ist, und damit in der Folge eine Verbreitung von Inhaltsstoffen des Klebers bis zur Haut des Babys, was häufig allergische Reaktionen auslöst.

Aufgrund der Faltung im unbenutzten Anlieferungszustand ergeben sich einerseits flache rechteckige, einfach und billig durch übliche Verpackungsanlagen steril zu verpackende Nabelverbände, und andererseits eine sehr einfache Handhabung, vor allem verglichen mit dem ansonsten benutzten aufgerollten Zustand unbehandelter Mullbinden.

Durch die Ausrichtung der Kontaktfläche der auf der Fixierbinde befestigten Kompresse in das Innere des Paketes hinein ist auch nach dem Herausnehmen des gefalteten Paketes aus der sterilen Umverpackung noch keine Kontaminationsgefahr für die Kontaktfläche der Kompresse gegeben.

Dadurch, daß über die beiden gegenüberliegenden Kanten der Kompresse - die vorzugsweise aus einem nicht mit einer Wunde verklebenden Vlies aufgrund nicht-freien Fasern besteht - auf der einen Seite ein relativ kurzes und auf der anderen Seite ein relativ langes Ende der Fixierbinde übersteht, ist es möglich, beim Anlegen des Nabelverbandes dieses kurze Ende festzuhalten, ohne dadurch die Kompresse selbst berühren zu müssen, und andererseits am angelegten langen Ende der Fixierbinde ziehen zu können, um die gewünschte Spannung in Längsrichtung einzustellen, ohne daß dadurch wiederum die Lage der Kompresse gegenüber dem Nabel verändert wird. Denn eine solche unter Druck stattfindende Verschiebung der Kompresse gegenüber dem Nabel ist schmerzhaft und kann auch einen bereits gebildeten Schorf wieder abreißen, und würde besonders dann zu befürchten stehen, wenn die Kompresse etwa in der Mitte der Länge der Fixierbinde angeordnet wäre, da es dann sehr schwierig wäre, beim Anlegen an den beiden gleich langen und damit einer beträchtlichen Dehnung unterliegenden Enden exakt gleichmäßig stark zu ziehen.

Das Anlegen des Nabelverbandes ohne Berührung der Kompresse durch die Hand des Benutzers wird besonders dann erleichtert, wenn das im ursprünglichen Zustand befindliche gefaltete Paket - welches eine Grundfläche nur noch entsprechend der Grundfläche der Kompresse besitzt - nur an dem über die Kompresse vorstehenden kurzen Ende ergriffen werden muß, was dadurch möglich ist, daß dieses kurze Ende auf die Rückseite der Kompresse geklappt ist und auch nicht von einer darüberliegenden, vollständigen Schicht abgedeckt ist.

Dabei ist das lange Ende lagenweise an der Kontaktfläche der Kompresse anliegend gefaltet, indem beispielsweise der sich an die Kompresse anschließende Längsbereich des langen Endes mit der Kompresse selbst ein U bildet, in dessen Freiraum ein V-förmig die sich an den ersten Längsbereich anschließenden beiden, zweiten und dritten Längsbereiche, hineingefaltet sind. Ein darüberhinaus überstehendes freies Ende des langen Bereiches der Fixierbinde ist um die Kompresse herum auf deren Rückseite geklappt und liegt damit auf dem kurzen Ende der Fixierbinde auf, mit dem es vorzugsweise gemeinsam endet.

Ein besonders gutes Handling ergibt sich, wenn in diesem gefalteten Zustand das Ende des langen Bereiches nicht ganz den Abschluß des kurzen Endes erreicht. Dadurch ist es möglich, durch Ergreifen des Haftstreifens, der über das Ende des langen Bereiches vorsteht, einerseits, und Ergreifen des darunter hervorragenden, kurzen Endes der Fixierbinde andererseits sofort die beiden Enden der Fixierbinden in der Hand zu haben, und damit ohne jede Berührung der Kompresse diese positionsgenau aufbringen zu können.

Erst nach dem Herumlegen des langen Endes um den Bauchumfang des Babys und dem Kontakt der beiden Enden in der richtigen Spannung muß dann die Schutzabdeckung vom Haftstreifen, der über das lange Ende vorsteht, entfernt werden, um den Nabelverband endgültig zu fixieren.

Dadurch, daß die als Fixierbinde benutzte Mullbinde nicht auf Breite geschnitten, sondern auf die gewünschte Breite gewebt ist, ergeben sich über die gesamte Breite der Mullbinde auch im wesentlichen die gleichen Zugspannungen, so daß auch die Kompresse über ihre gesamte Breite mit etwa dem gleichen Anpreßdruck auf den Nabel drückt.

Eine Ausführungsform gemäß der Erfindung ist im folgenden anhand der Figuren beispielhaft näher beschrieben. Es zeigen:
- Fig. 1: einen unbenutzten Nabelverband in der Seitenansicht und
- Fig. 2: das Öffnen eines unbenutzten Nabelverbandes.

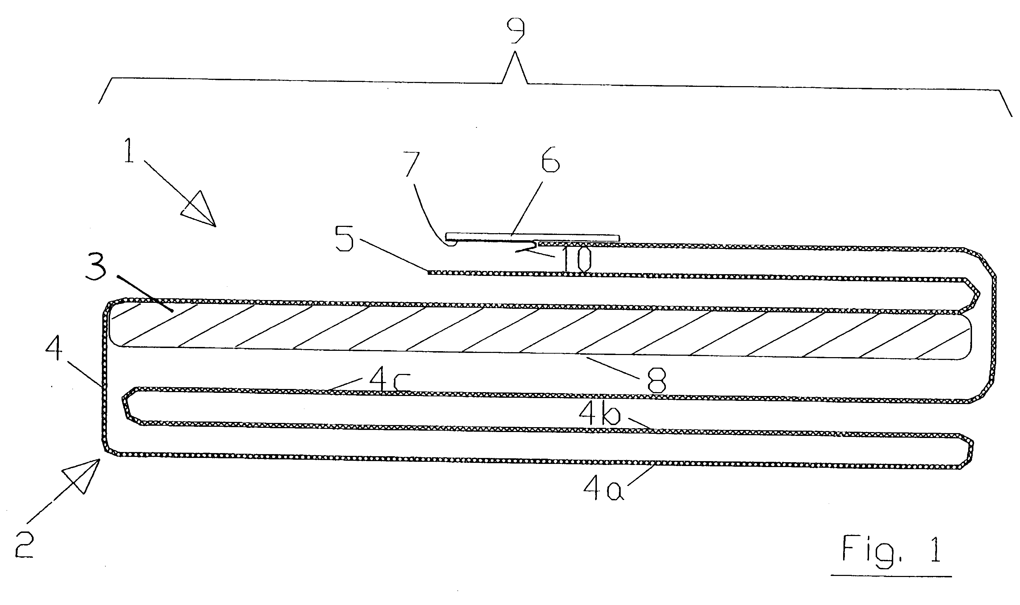

In Fig. 1 ist der noch unbenutzte, gefaltete Nabelverband in der Seitenansicht dargestellt, wie er aus seiner sterilen Umverpackung entnommen wird.

Die nicht sichtbare Breite des Nabelverbandes ist über die gesamte Länge im wesentlichen gleich, und auch die Breite des auf der Fixierbinde 2 des Nabelverbandes 1 fest angeordneten Kompresse 3 entspricht der Breite der Fixierbinde 2.

Vorzugsweise wird die Kompresse 3 auf der Fixierbinde 2 ohne Verwendung eines Klebstoffes befestigt, beispielsweise durch Verschweißen, indem das Vlies der Kompresse 3 thermoplastische Bestandteile enthält, die bei Erhitzung ein Verschweißen bzw. Verkleben beim Andrücken an die Fixierbinde 2 ergeben.

In der Darstellung der Fig. 1 ist die Kompresse 3 waagerecht angeordnet mit nach unten weisender Wundauflagefläche 8. Auf der obenliegenden Rückseite der Kompresse 3 ist sie mit der Fixierbinde 2 verbunden.

Dabei ragt über die rechte Kante der Kompresse 3 das kurze Ende 5 der Fixierbinde 2 hinaus, welches auf die Rückseite der Kompresse 3 umgeklappt ist, und in der Länge kürzer ist als die gesamte Länge der Kompresse 3. In der Praxis ist das kurze Ende etwa 4 bis 5 cm lang, während die Kompresse etwa 8 cm lang ist, und die Fixierbinde insgesamt eine Länge von etwa 45 cm (ungedehnt) besitzt bei einer Breite von etwa 6 cm.

Das lange Ende 4, welches über den linken Rand der Kompresse 3 übersteht, besitzt eine Länge entsprechend der etwa 3,5-fachen Länge der Kompresse 3. Anschließend an die Kompresse ergeben sich dadurch drei aneinander anschließende Längsbereiche 4a, 4b, 4c, die jeweils die einfache Länge der Kompresse 3 besitzen.

Dabei ist der erste, sich an die Kompresse anschließende, Längsbereich 4a gegen die Unterseite, die Wundauflagefläche 8 der Kompresse 3 gefaltet, wobei jedoch die nächsten beiden daran anschließenden Längsbereiche 4b, 4c V-förmig gefaltet in den Bereich zwischen den ersten Längsbereich 4a und die Kompresse 3 hineingefaltet sind.

Das an den dritten Längsbereich 4c anschließende freie Ende dieses langen Endes 5 ist dann um die Kompresse herum geschlagen und liegt auf dessen Rückseite, und damit auf dem dort angeordneten kurzen Ende 5 der Fixierbinde auf.

Dadurch ergibt sich eine M-förmige Faltung des langen Endes 4, in dessen erster Ausbuchtung die Kompresse 3 mit dem kurzen Ende 5 der Fixierbinde 2 liegt.

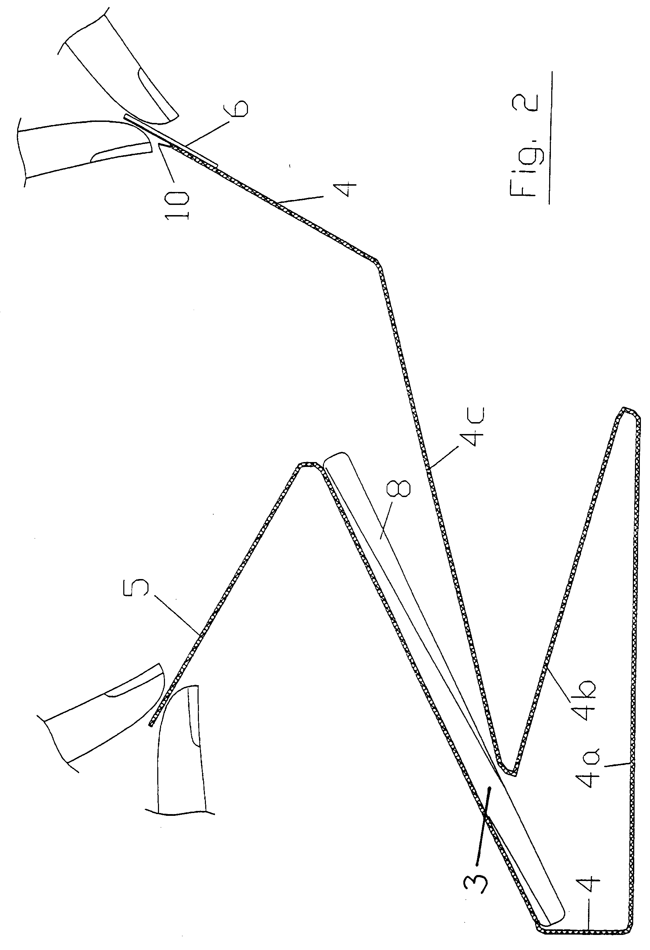

Fig. 1 zeigt bezüglich der Enden des langen sowie kurzen Endes 4 bzw. 5 den bevorzugten Zustand, daß das kurze Ende 5 noch unter dem langen Ende 4 hervorragt, jedoch nicht ganz die linke Kante der Kompresse 3 erreicht. Dadurch können beide Enden, sowohl die des langen als auch die des kurzen Bereiches der Fixierbinde, gleichzeitig und gleichgut ergriffen werden, wobei das lange Ende 4 entweder direkt an der Mullbinde oder an dem daran verklebten Haftstreifen 6 ergriffen werden kann, wie besser in Fig. 2 zu erkennen.

Denn nach dem Ergreifen dieser beiden Enden mit den Fingern jeweils einer Hand können diese auseinanderbewegt und vor allem durch Bewegen derjenigen Hand, die das kurze Ende ergriffen hat, die Kompresse 3 ohne vorherige Berührung auf dem Nabel des Babys positioniert werden.

Wie ferner in Figur 1 dargestellt, ist der über das lange Ende 4 hinausragende Teil des Haftstreifens 6, der meist ein herkömmlicher Klebestreifen über einem Heftpflaster sein wird, im über die Fixierbinde 2 hinausragenden Teil auf seiner Klebeschicht mit einer Schutzabdeckung 7 versehen, die ihrerseits wiederum an dem zur Fixierbinde 2 hin benachbarten Ende ein gekröpftes, überstehendes freies Ende 10 aufweist, welches vor der Befestigung des Haftstreifens leicht ergriffen und abgezogen werden kann.

Wie Fig. 1 zeigt, ergibt sich durch diese Faltung nicht nur eine einfache Handhabung, sondern im gefalteten Zustand auch ein sehr kompaktes Paket, dessen Grundfläche der Grundfläche der Kompresse 3 entspricht, und welches auch in der Dicke nur geringfügig dicker ist als die Kompresse 3, da die einzelnen Lagen der Fixierbinde 2 sehr dünn sind und eng aneinander anliegen, im Gegensatz zu der aus Übersichtlichkeitsgründen in der Dicke auseinandergezogenen Darstellung der Fig. 1.

## Patentansprüche

1. Nabelverband für Neugeborene mit einer Binde und einer über einen Teil der Länge der Binde auf dieser fest angeordneten Kompresse, **dadurch gekennzeichnet, daß**
- die Kompresse (3) in Längsrichtung des Nabelverbandes so auf der Fixierbinde (2) angeordnet ist, daß ein gegenüber der Kompresse (3) kurzes, überstehendes Ende (5) sowie ein demgegenüber wesentlich längeres Ende (4) der Fixierbinde (2) entstehen und
- über die Stirnseite des langen Endes (5) ein mit einer abziehbaren Schutzabdeckung (7) versehener Haftstreifen (6) hervorragt zur Verklebung auf dem kurzen Ende (5) der Fixierbinde (2) im applizierten Zustand.

2. Nabelverband nach Anspruch 1,
**dadurch gekennzeichnet, daß**
im unbenutzten Zustand der Nabelverband (1) so gefaltet ist, daß
- ein flaches Paket (9) entsteht, dessen Grundfläche im wesentlichen der Grundfläche der Kompresse (3) entspricht und
- die Wundauflagefläche (8) der Kompresse (3) in das Innere des Paketes (9) hineingerichtet ist.

3. Nabelverband nach einem der vorhergehenden Ansprüche,
**dadurch gekennzeichnet, daß**
die Fixierbinde (2) so lang ist, daß unter der für die Applikation notwendigen Spannung in Längsrichtung der über das lange Ende (4) vorstehende Haftstreifen (6) bereits das andere, kurze Ende (5) der Fixierbinde (2) erreicht und auf diesem vollständig befestigt werden kann und selbst bei Babys mit geringem Bauchumfang bei der Applizierung der über das lange Ende (4) vorstehende Haftstreifen (6) nicht im Bereich der Kompresse (3) zu liegen kommt.

4. Nabelverband nach einem der vorhergehenden Ansprüche,
**dadurch gekennzeichnet, daß**
die Kompresse (3) eine solche Länge hat, daß das lange Ende (4) der Fixierbinde (2) die drei- bis vierfache Länge und das kurze Ende (5) der Fixierbinde (2) maximal die einfache Länge der Kompresse (3) aufweist.

5. Nabelverband nach einem der vorhergehenden Ansprüche,
**dadurch gekennzeichnet, daß**
die Fixierbinde (2) aus in Längsrichtung elastischer Mullbinde, vorzugsweise mit hohem Baumwollanteil, besteht, die im ungedehnten Zustand etwa 45 cm lang ist, und die Kompresse eine Länge von etwa 8 cm besitzt.

6. Nabelverband nach einem der vorhergehenden Ansprüche,
**dadurch gekennzeichnet, daß**
im unbenutzten, gefalteten Zustand des Nabelverbandes das kurze Ende (4) der Fixierbinde (2) auf die Rückseite der Kompresse (3) geklappt ist und der an die Kompresse (3) anschliessende erste Längsbereich (4a) des langen Endes (4) auf die Wundauflagefläche (8) der Kompresse (3) geklappt wird, sowie die sich an den ersten Längsbereich (4a) anschließenden zweiten und dritten Längsbereiche (4b, 4c) des langen Endes (4) der Fixierbinde (2) in den Bereich zwischen den ersten Längsbereich 4a und die Kompresse 3 hineingefaltet sind, und das restliche, freie Ende des langen Endes (4) auf das auf der Rückseite der Kompresse (3) befindliche kurze Ende (5) geklappt ist.

7. Nabelverband nach einem der vorhergehenden Ansprüche,
**dadurch gekennzeichnet, daß**
das kurze und das lange Ende (4, 5) im gefalteten Zustand des Nabelverbandes (1) etwa auf gleicher Höhe auf der Rückseite der Kompresse (3) enden.

8. Nabelverband nach einem der vorhergehenden Ansprüche,
**dadurch gekennzeichnet, daß**
das kurze und das lange Ende (4, 5) im gefalteten Zustand des Nabelverbandes (1) etwa auf gleicher Höhe auf der Rückseite der Kompresse (3) enden, wobei das oben liegende, lange Ende (4) das Ende des unten liegenden, kurzen Endes (5) nicht erreicht.

9. Nabelverband nach einem der vorhergehenden Ansprüche,
**dadurch gekennzeichnet, daß**
die Kompresse (3) aus einem nicht mit einer Wunde verklebenden Vlies besteht.

10. Nabelverband nach einem der vorhergehenden Ansprüche,
**dadurch gekennzeichnet, daß**
die Kompresse (3) mit der Fixierbinde (2) mittels Erhitzen der Rückseite der Kompresse (3) verschweißt ist.

11. Nabelverband nach einem der vorhergehenden Ansprüche,
**dadurch gekennzeichnet, daß**
die Fixierbinde (2) an ihren Seitenrändern nicht geschnitten, sondern auf die passende Breite gewebt ist.

12. Nabelverband nach einem der vorhergehenden Ansprüche,
**dadurch gekennzeichnet, daß**
der Haftstreifen (6) ein einseitig mit Klebstoff beschichteter Klebestreifen ist, der mit seiner einen Hälfte auf dem stirnseitigen Ende des langen Endes (4) der Fixierbinde (2) verklebt ist und dessen andere Hälfte mit einer Schutzabdeckung (7) gesichert ist.

13. Nabelverband nach einem der vorhergehenden Ansprüche,
**dadurch gekennzeichnet, daß**
die Schutzabdeckung (7) ein zum Ende der Fixierbinde (2) und damit der Mitte des Haftstreifens (6) ein überstehendes, freies Ende (10) zum besseren Ergreifen aufweist.

## Claims

1. An umbilical dressing for newborn children comprising a bandage and a compress which is fixedly arranged on the bandage over a part of the length thereof, characterised in that
- the compress (3) is so arranged on the fixing bandage (2) in the longitudinal direction of the umbilical dressing that there are formed a projecting end (5) of the fixing bandage (2), which end is short in relation to the compress (3), and an end (4) of the fixing bandage (2), which is substantially longer in relation to the short end, and
- projecting beyond the end edge of the long end (6) is an adhesive strip (6) provided with a pull-off protective cover (7), for adhesively fastening on the short end (5) of the fixing bandage (2) in the applied condition.

2. An umbilical dressing according to claim 1 characterised in that in the unused condition the umbilical dressing (1) is so folded that
- there is formed a flat pack (9) whose surface in plan view substantially corresponds to the surface in plan view of the compress (3), and
- the wound-contacting surface (8) of the compress (3) is directed into the interior of the pack (9).

3. An umbilical dressing according to one of the preceding claims characterised in that the fixing bandage (2) is so long that, under the necessary stress for application in the longitudinal direction the adhesive strip (6) which projects beyond the long end (4) already reaches the other short end (5) of the fixing bandage (2) and can be completely secured thereon and the adhesive strip (6) which projects beyond the long end (4) does not come to lie in the region of the compress (3) even in relation to babies of a small tummy girth when the dressing is applied.

4. An umbilical dressing according to one of the preceding claims characterised in that the compress (3) is of such a length that the long end (4) of the fixing bandage (2) is of three to four times the length of the compress and the short end (5) of the fixing bandage (2) is at a maximum just of the length of the compress (3).

5. An umbilical dressing according to one of the preceding claims characterised in that the fixing bandage (2) comprises gauze bandage which is elastic in the longitudinal direction, preferably with a high cotton content, which in the unstretched condition is about 45 cm long, and the compress is about 8 cm in length.

6. An umbilical dressing according to one of the preceding claims characterised in that in the unused folded condition of the umbilical dressing the short end (4) of the fixing bandage (2) is folded on to the rear side of the compress (3) and the first lengthwise region (4a) of the long end (4), which region adjoins the compress (3), is folded on to the wound-contacting surface (8) of the compress (3) and the second and third lengthwise regions (4b, 4c) of the long end (4) of the fixing bandage (2), said second and third regions adjoining the first lengthwise region (4a), are folded into the region between the first lengthwise region (4a) and the compress, and the remaining free end of the long end (4) is folded on to the short end (5) which is on the rear side of the compress (3).

7. An umbilical dressing according to one of the preceding claims characterised in that in the folded condition of the umbilical dressing (1) the short and the long ends (4, 5) end approximately at the same level on the rear side of the compress (3).

8. An umbilical dressing according to one of the preceding claims characterised in that in the folded condition of the umbilical dressing (1) the short and the long ends (4, 5) end approximately at the same level on the rear side of the compress (3), the long end (4) which is on top not reaching the end of the short end (5) which is on the bottom.

9. An umbilical dressing according to one of the preceding claims characterised in that the compress (3) comprises a fleece material which does not stick to a wound.

10. An umbilical dressing according to one of the preceding claims characterised in that the compress (3) is welded to the fixing bandage (2) by means of heating of the rear side of the compress (3).

11. An umbilical dressing according to one of the preceding claims characterised in that the fixing bandage (2) is not cut at its side edges but is woven to the appropriate width.

12. An umbilical dressing according to one of the preceding claims characterised in that the adhesive strip (6) is a glueing strip which is coated with adhesive on one side and which is glued with its one half to the end part of the long end (4) of the fixing bandage (2) and the other half of which is protected by a protective cover (7).

13. An umbilical dressing according to one of the preceding claims characterised in that at the end of the fixing bandage (2) and thus the middle of the adhesive strip (6) the protective cover (7) has a projecting free end (10) for better gripping same.

## Revendications

1. Pansement ombilical pour nouveau-nés avec un bandage et une compresse disposée fixement sur une partie de la longueur du bandage, caractérisé en ce que
- la compresse (3) est disposée dans le sens longitudinal du pansement ombilical sur le bandage de fixation (2), de manière à former une extrémité 5 saillante et courte par rapport à la compresse (3) et une extrémité (4) sensiblement plus longue du bandage de fixation (2) et
- sur la face avant de l'extrémité longue (5) dépasse une bande adhésive (6) munie d'un revêtement de protection (7) pouvant être retiré en vue du collage sur l'extrémité courte (5) du bandage de fixation (2) à l'état appliqué.

2. Pansement ombilical selon la revendication 1, caractérisé en ce qu'à l'état non utilisé, le pansement ombilical (1) est plié de sorte
- qu'on obtient un paquet plat (9) dont la surface de base correspond sensiblement à la surface de base de la compresse (3) et
- la surface d'application (8) contre la blessure de la compresse (3) est dirigée vers l'intérieur du paquet (9).

3. Pansement ombilical selon l'une des revendications précédentes, caractérisé en ce que le bandage de fixation (2) est suffisamment long pour que sous la tension nécessaire à l'application dans le sens longitudinal, la bande adhésive (6) dépassant de l'extrémité longue (4) atteigne déjà l'autre extrémité courte (5) du bandage de fixation (2) et puisse être fixée complètement sur cette dernière et que même pour des nourrissons avec un petit ventre, la bande adhésive (6) dépassant de l'extrémité longue (4) ne vienne pas s'appliquer dans la zone de la compresse (3) au moment de la mise en place.

4. Pansement selon l'une des revendications précédentes, caractérisé en ce que la longueur de la compresse (3) est telle que l'extrémité longue (4) du bandage de fixation (2) fait le triple au quadruple de la longueur de la compresse (3) et l'extrémité courte (5) du bandage de fixation (2) représente au maximum une fois la longueur de cette dernière.

5. Pansement selon l'une des revendications précédentes, caractérisé en ce que le bandage de fixation (2) se compose d'une bande de gaze élastique dans le sens de la longueur de préférence avec une teneur en coton élevée qui, à l'état non étiré, mesure environ 45 cm de long et en ce que la compresse mesure environ 8 cm de long.

6. Pansement selon l'une des revendications précédentes, caractérisé en ce qu'à l'état plié non utilisé du pansement ombilical, l'extrémité courte (4) du bandage de fixation (2) est rabattue sur l'envers de la compresse (3) et la première zone longitudinale (4a)de l'extrémité longue (4) se trouvant dans le prolongement de la compresse (3) est rabattue sur la surface d'application (8) de la compresse contre la blessure ainsi que les seconde et troisième zones longitudinales (4b, 4c) de l'extrémité longue (4) du bandage de fixation (2), lesquelles se trouvent dans le prolongement de la première zone longitudinale (4a) sont pliées à l'intérieur de la zone se trouvant entre la première zone longitudinale (4a) et la compresse (3) et le bout restant de l'extrémité libre longue (4) est rabattu sur l'extrémité courte (5) se trouvant sur l'envers de la compresse (3).

7. Pansement selon l'une des revendications précédentes, caractérisé en ce que l'extrémité courte et l'extrémité longue (4, 5) se terminent à l'état plié du pansement ombilical (1) à peu près au même niveau sur l'envers de la compresse (3).

8. Pansement selon l'une des revendications précédentes, caractérisé en ce que l'extrémité courte et l'extrémité longue (4, 5) se terminent à l'état plié du pansement ombilical (1) à peu près au même niveau sur l'envers de la compresse (3), l'extrémité longue (4) se trouvant an haut n'atteignant pas le bout de l'extrémité courte (5) se trouvant en bas.

9. Pansement selon l'une des revendications précédentes, caractérisé en ce que la compresse (3) se compose d'un voile ne collant pas à la plaie.

10. Pansement selon l'une des revendications précédentes, caractérisé en ce que la compresse (3) est thermoscellée au bandage de fixation (2) en chauffant l'envers de la compresse (3).

11. Pansement selon l'une des revendications précédentes, caractérisé en ce que le bandage de fixation (2) n'est pas coupé sur ses bords latéraux mais tissé à la largeur appropriée.

12. Pansement selon l'une des revendications précédentes, caractérisé en ce que le ruban adhésif (6) est un ruban collant revêtu d'une colle sur une face, qui est collé par une moitié sur le bout avant de l'extrémité longue (4) du bandage de fixation (2) et dont l'autre moitié est maintenue par un revêtement de protection (7).

13. Pansement selon l'une des revendications précédentes, caractérisé en ce que le revêtement de protection (7) présente une extrémité libre (10), dépassant en direction de l'extrémité du bandage de fixation (2) et ainsi en direction du milieu du ruban adhésif (6).
